Europäisches Patentamt

European Patent Office

Office européen des brevets

(19)

(11) Publication number: **0 167 162**
**B1**

(12) **EUROPEAN PATENT SPECIFICATION**

(45) Date of publication of patent specification: **23.11.89**

(51) Int. Cl.⁴: **A 61 M 1/14**

(21) Application number: **85108287.5**

(22) Date of filing: **04.07.85**

(54) Hollow fiber type oxygenator.

(30) Priority: **04.07.84 JP 138695/84**

(43) Date of publication of application:
**08.01.86 Bulletin 86/02**

(45) Publication of the grant of the patent:
**23.11.89 Bulletin 89/47**

(84) Designated Contracting States:
**DE FR GB IT NL SE**

(56) References cited:
**EP-A-0 005 866**
**EP-A-0 103 899**

(73) Proprietor: **TERUMO KABUSHIKI KAISHA
trading as TERUMO CORPORATION
44-1, 2-chome, Hatagaya Shibuya-Ku
Tokyo 151 (JP)**

(72) Inventor: **Deguchi, Hiromi
3-209, Johoku
Shizuoka-shi Shizuoka (JP)**
Inventor: **Nagayama, Kiyotaka
12-58-2-403, Uke 1-chome Unoke-machi
Kahoku-gun Ishikawa (JP)**

(74) Representative: **Henkel, Feiler, Hänzel & Partner
Möhlstrasse 37
D-8000 München 80 (DE)**

Courier Press, Leamington Spa, England.

## Description

The present invention relates to a hollow fiber type oxygenator of the kind defined by the precharacterizing features of claim 1.

An oxygenator of this kind is known from the EP—A—103 899. The bundle of hollow fibers of this known oxygenator has a packing density $d_1$ of 20 to 50% near the partitions and a packing density $d_2$ of 24 to 80% near an axial midpoint. Gravity drainage of blood flowing from the patient to the oxygenator without positive aid is not ensured over the given ranges of the packing densities together with sufficient oxygen exchange capacity. In other words there are values within the given ranges that ensure either gravity drainage or sufficient oxygen exchange without ensuring the combination of both effects.

It is therefore the object of the invention to provide an improved hollow fiber type oxygenator of the kind defined at the preamble which ensures sufficient oxygen exchange capacity as well as gravity drainage from the patient to the oxygenator.

This object is attained by the characterizing features of claim 1. Advantageous developments of the invention are given by the features of the subclaims.

In accordance with the invention the bundle of hollow fibers has a packing density $d_1$ of 20 to 30% near the partitions and a packing density $d_2$ of 40 to 50% near an axial midpoint, the ratio of $d_1/d_2$ ranging from 4/10 to 6/10. It is the combination of these three requirements that ensure that the oxygenator has a sufficient oxygen exchange capacity and simultaneously causes a pressure loss of not more than 60 mmHg or less, so that blood can flow from the patient to the oxygenator under gravity without positive aid.

The invention too is of advantage insofar as the oxygenator housing is not unnecessarily increased in size, thereby offering a relatively compact oxygenator requiring a relatively reduced volume of the priming blood.

## Brief description of the drawings

These and other objects, features, and advantages of the present invention will be readily understood from the following description taken in conjunction with the accompanying drawings, in which:

Fig. 1 is an elevational cross section of a hollow fiber type oxygenator according to one embodiment of the present invention;

Figs. 2, 3 and 4 illustrates different circuit arrangements wherein the oxygenator of the present invention is used;

Fig. 5 is a diagram showing the volume of oxygen migrated and the pressure loss ΔP in relation to the end packing density $d_1$;

Fig. 6 is a diagram showing the volume of oxygen migrated and the pressure loss ΔP in relation to the central packing density $d_2$;

Fig. 7 is a diagram showing the volume of oxygen migrated and the pressure loss ΔP in relation to the ratio of $d_1/d_2$;

Fig. 8 is an elevational cross section of a hollow fiber type oxygenator according to another embodiment of the present invention; and

Fig. 9 is a perspective view of a hollow fiber type oxygenator similar to that shown in Fig. 8.

## Description of the preferred embodiments

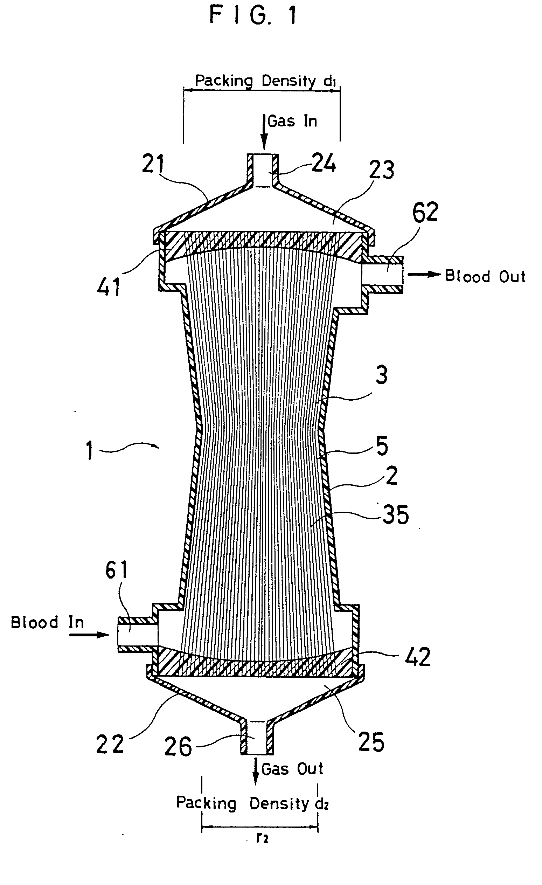

Referring to Fig. 1, there is schematically illustrated a hollow fiber type oxygenator according to one embodiment of the present invention. The oxygenator generally designated at 1 includes a hollow cylindrical housing 2 defining an interior space and being open at axially opposite end portions. The housing 2 receives therein a bundle or assembly 35 of a plurality of axially extending hollow fibers 3 each presenting a gas exchange membrane. The opposite ends of the hollow fibers 3 are fluid tightly retained by opposed partitions 41 and 42 which are engaged in the housing 2 at its opposite ends, respectively. For brevity of description, the partitions 41 and 42 and the corresponding housing ends are referred to as upper and lower partitions and upper and lower housing ends.

Upper and lower conical headers 21 and 22 are fixedly secured to the upper and lower ends of the housing 2. The inner surface of the upper header 21 and the outer surface of the upper partition 41 define a gas incoming chamber 23 in fluid communication with the interior space of hollow fibers 3. The header 21 has a gas inlet 24 at its apex. The inner surface of the lower header 22 and the outer surface of the lower partition 42 define a gas outgoing chamber 25 in fluid communication with the interior space of hollow fibers 3. The header 22 has a gas outlet 26 at its apex.

In the oxygenator 1 of this arrangement, a gas such as oxygen and air can be passed from the inlet 24 through the interior space of the hollow fibers 5 in the outlet 26.

The lower header 22 may not necessarily be provided, that is, the outgoing chamber 25 and outlet 26 may be omitted. Then the outer side of the lower partition 42 becomes a gas discharge end and the gas coming out of the hollow fibers 3 is directly released into the atmosphere.

A blood chamber 5 is defined by the inner surfaces of the partitions 41, 42, the inner wall of the housing 2, and outer wall of the hollow fibers 3. The housing 2 is provided near its lower and upper ends with a blood inlet 61 and a blood outlet 62 both in fluid communication with the blood chamber 5. The oxygenator permits blood to pass through the blood chamber 5 in a turbulent flow around hollow fiber membranes 3.

That portion of the wall of the housing 2 where the blood inlet 61 is provided is radially dilated as compared with the intermediate portion. An annular space is thus defined between the outer periphery of the bundle 35 of hollow fibers 3 and the radially dilated housing wall, providing an annular blood path. The annular flow path circumscribes the bundle 35 of hollow fibers 3 so that blood is smoothly and evenly distributed among all the hollow fibers 3. Also, that portion of the wall of the housing 2 where the blood outlet 62 is provided is radially dilated as compared with the intermediate portion. An annular space is thus defined between the outer periphery of the bundle 35 of hollow fibers 3 and the radially dilated housing wall, providing an annular blood flow path. The annular flow path circumscribes the bundle 35 of hollow fibers 3 so that blood flowing around the hollow fibers 3 is smoothly directed to the blood outlet 62 from the entire periphery of the fiber bundle 35.

The wall of the housing 2 is tapered toward an axial mid-point. The housing wall has a minimum inner diameter approximately at the mid-point and is flared or divergent therefrom toward the upper and lower ends.

The wall of the housing 2 is tapered toward the mid-point to squeeze or throttle the bundle or assembly 35 of hollow fibers at the axial mid-point. The throttling of the fiber assembly 35 by the housing 2 ensures a uniform blood flow in a transverse cross-section of the assembly 35 and changes the flow velocity of blood in axial direction of the fiber assembly 35, promoting the occurrence of turbulent flow to improve the gas exchangeability of the oxygenator.

In addition to the tapering of the housing wall toward the mid-point as previously described, preferably the tapered wall of the housing 2 is connected to the radially dilated wall defining the annular blood flow path by a tapered surface (not shown). Then air to be purged upon priming is smoothly driven off along the inner surface of the housing 2 and released into the atmosphere without remaining in the blood chamber 5.

The hollow fiber membranes 3 used in the oxygenator of the invention may be microporous membranes. The porous hollow fibers may preferably be formed of a polyolefin such as polypropylene and polyethylene. Polypropylene is most preferred. Each hollow fiber (membrane) used in the present invention has many micropores which communicate the interior and the exterior of the membrane. The fiber has an inner diameter of about 100 to about 1,000 μm, a wall thickness of about 10 to 200 μm, an average pore diameter of about 200 to about 2,000 Å, and porosity of 20 to 80%.

When the hollow fibers 3 of the microporous membrane type are used, gas migration takes place as a volume flow. The resistance of the membrane to gas migration is reduced and the gas exchangeability of the membrane is improved.

Silicone membranes which provide for gas migration through disolution and diffusion process may also be employed as well as the microporous membranes.

The partitions 41 and 42 serve for the important function of isolating the interior from the exterior of the hollow fibers 3. In general, the partitions 41 and 42 are formed by casting a highly polar, high molecular weight potting compound, for example, polyurethane, silicone and epoxy resins by a centrifugal casting process to the inner wall surface of the housing end portions where it is cured.

More illustratively, first a number of hollow fibers 3 longer than the axial length of the housing 2 are prepared, opposite open ends of the fibers are sealed with a viscous resin, and then the fibers are placed in the housing 2 in mutually juxtaposed relationship. The opposite ends of the fiber bundle are completely covered. The housing 2 is rotated about its longitudinal axis while a polymeric potting compound is introduced from the sides of blood inlet 61 and outlet 62 where it is cured. The edge portions of the fiber bundle bonded with the potting compound are severed with a sharp knife to expose freshly cut openings of the hollow fibers 3 at their opposite ends. The partitions 41 and 42 are thus formed. Those surfaces of the partitions 41 and 42 facing the blood chamber 5 are circular and concave as shown in Fig. 1.

In order that the oxygenator thus prepared may exert satisfactory performance, the bundle 35 of hollow fibers must meet certain limitations with respect to fiber packing density. First, the hollow fiber bundle 35 must have a packing density $d_1$ of not more than 30% at the location of partitions 41 and 42. The term packing density used in the present invention is the sum of the outer diameter areas of the hollow fibers 3 divided by the area defined by the envelope of the fiber bundle 35 as expressed in percentage.

With a packing density $d_1$ of above 30%, the amount of oxygen permeated through the membranes is reduced below a practically required level. Also an increased pressure loss prevents passage of blood by gravity.

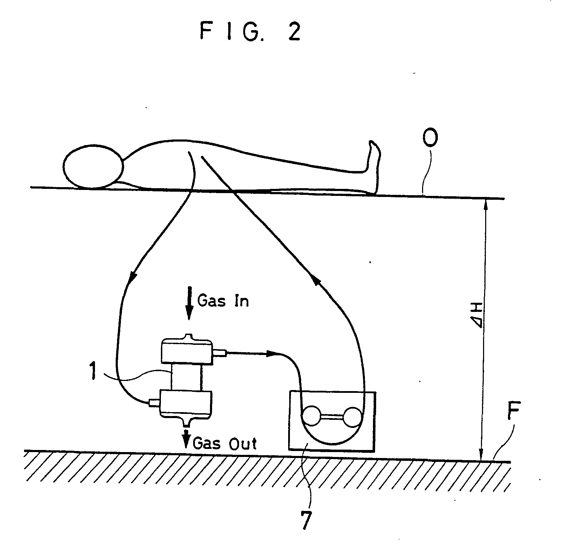

More illustratively, a head ΔH of 90 to 120 cm, preferably 100 cm, is set between an operating table O and a floor level F in order to enable passage of blood by gravity as shown in Fig. 2. This means that the passage of blood by gravity may not be conducted unless the pressure loss is kept less than 60 mmHg.

The oxygenator must accommodate a maximum blood flow rate of approximately 6.0 l/min. The oxygenator must have such an oxygenating capacity that the amount of oxygen migrated through the membranes be at least 240 ml/min, at a blood flow rate of 6.0 l/min.

These requirements are satisfied when the packing density $d_1$ is equal to or less than 30%. More preferable results are obtained when the packing desnity $d_1$ is from 20% to 30%. When the packing density $d_1$ is less than 20%, the oxygenator is no longer compact and undesirably increases the volume of blood contained therein.

Secondly, the hollow fiber bundle 35 must have a packing density $d_2$ of 40% to 50% near its axial mid-point or the throat of the housing 2.

A packing density $d_2$ of more than 50% makes impossible the passage of blood by the head or

gravity, whereas a packing density $d_2$ of less than 40% produces a practically unacceptable oxygenating capacity.

The ratio of fiber packing density $d_1/d_2$ should be kept equal to or below 0.6. Ratios of $d_1/d_2$ in excess of 0.6 make impossible the passage of blood by the head or gravity and result in a low oxygenating capacity. For the compactness of the oxygenator, the ratio of $d_1/d_2$ may preferably be between 0.4 and 0.6.

Further, the radius $r_2$ of the hollow fiber bundle 35 at the housing throat may preferably be not less than 60 mm, when the blood flow rate $Q_B$ is 6.0 l/min. Then the blood flow rate solely caused by the head is increased. For compactness purpose, $r_2$ may preferably be less than about 90 mm.

In order to obtain an improved oxygenating capacity, it is desirable that the total membrane surface area may preferably be at least 1.5 square meter as calculated on the basis of the inner diameter of the hollow fibers. A total membrane area of about 2.0 to 3.0 $m^2$ is recommended for compactness.

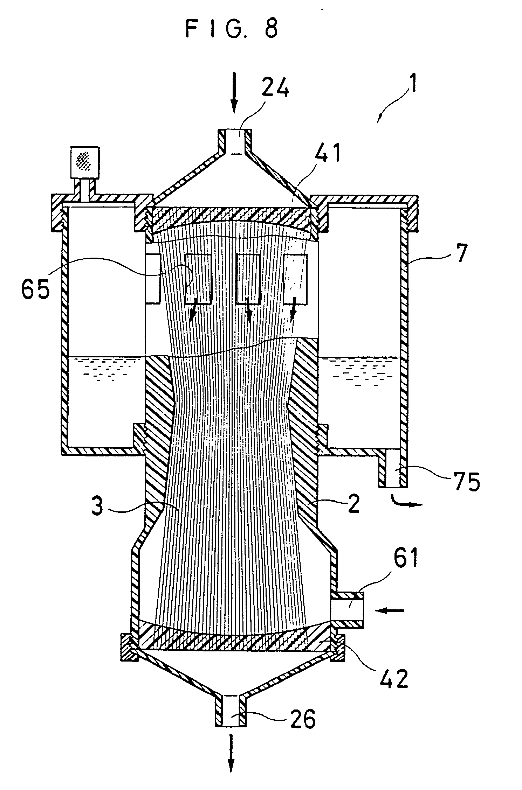

Another embodiment of the hollow fiber type oxygenator according to the present invention is shown in Fig. 8. Fig. 8 shows an oxygenator similar to the previously described oxygenator shown in Fig. 1 with the exception that the blood outlet 62 is replaced with blood drain means in the form of a plurality of blood drain openings 65 and blood reserving chamber 7 is further provided in fluid communication with the blood drain means. The blood reserving chamber 7 is provided at the bottom with an outlet 75.

The oxygenator 1 shown in Fig. 8 permits the line between the blood outlet and a blood reservoir to be omitted so as to reduce the volume of blood retained in the overall circuit. This is particularly effective when the oxygenator is used for a neonate with a low absolute volume of blood. Another advantage is ease of handling due to simplified circuit.

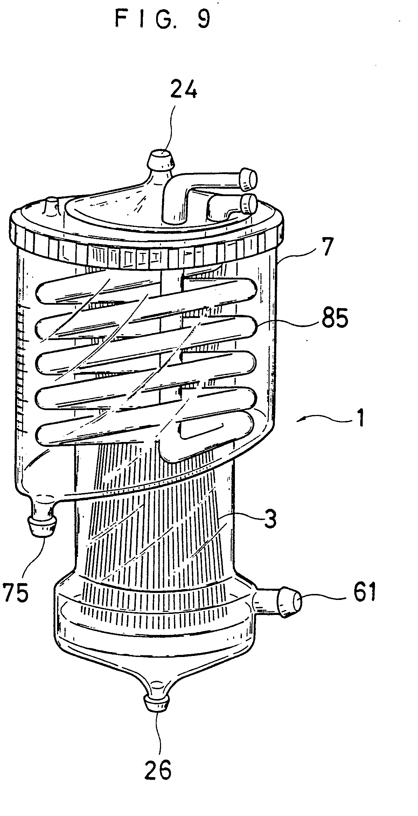

As shown in Fig. 9, the blood reserving chamber 7 may be provided with a heat exchanger 85, with the advantage of omitting a line connecting the blood storage chamber to a heat exchanger.

Effect of the invention

Fig. 2 shows a circuit arrangement wherein the oxygenator 1 of the invention is used in combination with a roller pump 7 placed downstream of the oxygenator, whereby blood is passed through the oxygenator by gravity or the head between the patient and the oxygenator.

A pressure loss of less than 60 mmHg is achieved with the present oxygenator at a head $\Delta H$ of approximately 100 cm so that a sufficient blood flow rate is secured. The amount of oxygen migrated can be at least 240 l/min. at a blood flow rate of 6.0 l/min. The oxygenator of the invention has another benefit over prior art oxygenators of the type wherein blood is passed inside hollow fiber membranes, that an equivalent oxygenating capacity is obtained with an about one half membrane area, which results in size, weight and cost reductions.

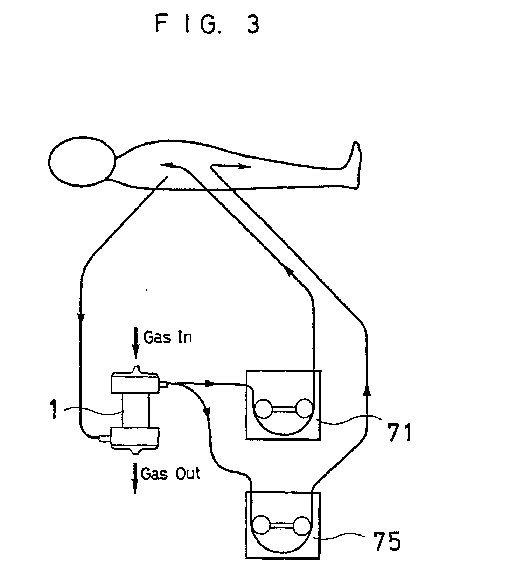

Fig. 3 shows another circuit arrangement wherein the oxygenator 1 of the invention is used in combination with two roller pumps 71 and 75 placed downstream of the oxygenator. Separate extracorporeal circulation can be smoothly carried out, because of the capacity of the oxygenator.

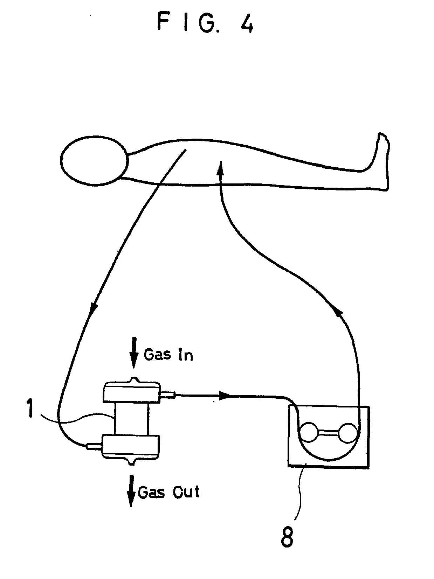

Fig. 4 shows a further circuit arrangement wherein the oxygenator 1 of the invention is used in combination with a pulsatile pump 8.

According to embodiments (i) and (ii), the size of the oxygenator is reduced and the volume of blood retained therein is accordingly reduced. An oxygenator according to embodiment (iii) achieves a remarkably high gas exchangeability. An oxygenator according to embodiment (iv) offers a uniform flow of blood resulting in an improved gas exchangeability.

The oxygenator thus constructed was tested in confirm the effect of the invention. One of such experiments is described hereinafter.

Example

An oxygenator as shown in Fig. 1 was manufactured using hollow microporous fibers of polypropylene which were formed by axially stretching to the following specifications: length 85 mm, inner diameter 200 μm, outer diameter 240 μm, average pore diameter 650 Å, and porosity 45%. The fiber bundle had a total membrane area of 2.5 $m^2$ as calculated on the basis of the inner diameter of hollow fibers, and a radius $r_2$ at the throat of 76 mm.

The fiber bundles having varying fiber packing densities $d_1$ and $d_2$ were assembled in housings. Bovine blood having a Hematocrit value Ht of 35% at 37°C was passed through the oxygenators at a blood flow rate $Q_B$ of 6 l/min., while oxygen gas was passed at a flow rate V of 6 l/min. As described above, the lower limit of the volume of oxygen migrated through the membranes is 240 ml/min. and the upper limit of the pressure loss $\Delta P$ during passage of blood by gravity is 60 mmHg.

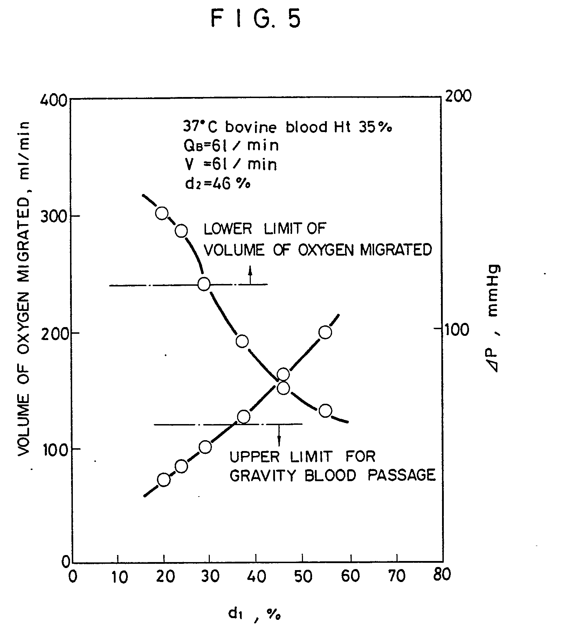

Fig. 5 shows the volume of oxygen migrated and the pressure loss $\Delta P$ in relation to the end packing density $d_1$ when the central packing density $d_2$ is set to 46%. The end packing density $d_1$ could be 30% or less in order to clear the critical points.

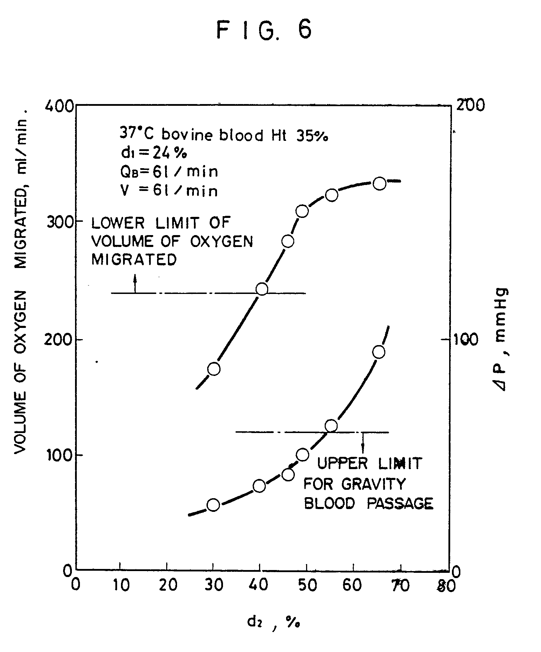

Fig. 6 shows the volume of oxygen migrated and the pressure loss $\Delta P$ in relation to the central packing density $d_2$ when the end packing density $d_1$ is set to 24%. The central packing density $d_2$ should range between 40% and 50% (about 53% under the experimented conditions).

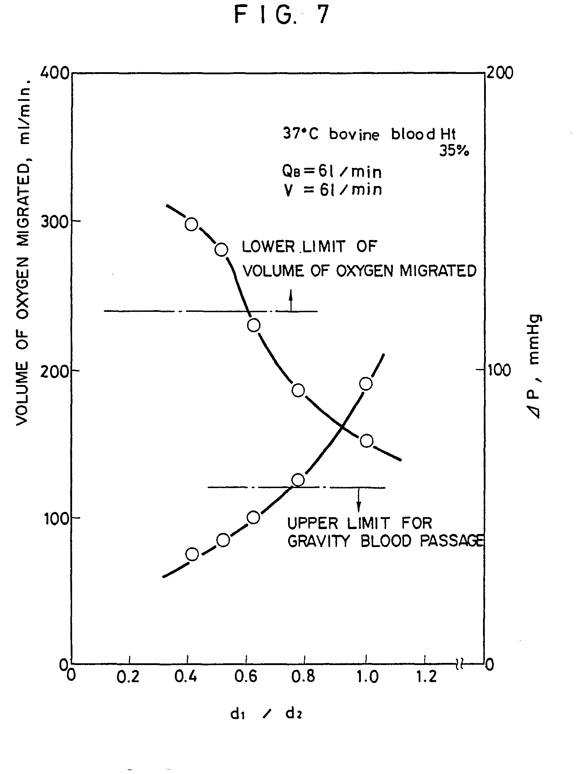

Fig. 7 shows the volume of oxygen migrated and the pressure loss $\Delta P$ in relation to the ratio of packing density $d_1/d_2$. The ratio $d_1/d_2$ should be 0.6 or less.

The data in Figs. 5, 6 and 7 reveal that the packing densities defined in the present invention are critical.

## Claims

1. A hollow fiber type oxygenator (1) comprising:

a housing (2) having opposite end portions,

a bundle (35) of a plurality of hollow fibers (3) axially extending through the housing (2) and each presenting a gas-exchange membrane,

partitions (41, 42) engaged in the housing (2) at its opposite end portions and fluid tightly retaining the opposite ends of the hollow fibers (3) without blocking the opening of the fibers (3),

a gas inlet (24) and a gas outlet (26) both in fluid communication with the interior space of said hollow fibers (3),

a blood chamber (5) defined by the portions (41, 42), the outer surface of said hollow fibers (3) and the inner surface of said housing (2), and

a blood inlet (61) and a blood outlet (62) located in the housing wall at the opposite end portions (41, 42) in fluid communication with said blood chamber (5), characterized in that

the bundle (35) of hollow fibers (3) has a packing density $d_1$ of 20 to 30% near the partitions (41, 42) and a packing density $d_2$ of 40 to 50% near an axial mid-point, the ratio $d_1/d_2$ ranging from 4/10 to 6/10, thereby limiting the pressure loss during passage of blood by gravity up to 60 mmHg.

2. The oxygenator (1) according to claim 1, wherein each of said hollow fibers (3) is a porous hollow fiber made of a polyolefin having an inner diameter of about 100 to about 1,000 µm, a wall thickness of about 10 to about 200 µm, an average pore diameter of about 200 to about 2,000 Å and a porosity of 20 to 80%.

3. The oxygenator (1) according to claim 2, wherein the polyolefin is polyproplene.

4. The oxygenator (1) according to claim 1, wherein the wall of the housing (1) is tapered toward an axial mid-point such that the wall thickness has a minimum inner diameter approxiamtely at the mid-point and is flared therefrom toward the opposite end portions.

## Patentansprüche

1. Hohlfaseroxygenator (1) mit:

einem gegnüberliegende Endabschnitte aufweisenden Gehäuse (2),

einen Bündel (35) einer Mehrzahl von Hohlfasern (3), die in axialer Richtung durch das Gehäuse (2) verlaufen, und von denen jede eine Gasaustauschmembran darstellt,

Trennwänden (41, 42), die in das Gehäuse (2) an dessen gegenüberliegenden Endabschnitten eingreifen und die gegenüberliegenden Enden der Hohlfasern (3) fluiddicht aufnehmen, ohne die Öffnungen der Fasern (3) zu blockieren,

einem Gaseinlaß (24) und einem Gasauslaß (26), die beide in Fluidübertragungsverbindung mit dem Innenraum der Hohlfasern (3) stehen,

einer Blutkammer (5), welche durch die Trennwände (41, 42), die Außenfläche der Hohlfasern (3) und die Innenfläche des Gehäuses (2) bestimmt ist, und einem Bluteinlaß (61) sowie einem Blutauslaß (62), die in der Gehäusewandung an den gegenüberliegenden Endabschnitten (41, 42) in Fluidübertragungsverbindung mit der Blutkammer (45) angeordnet sind, dadurch gekennzeichnet, daß

das Bündel (35) der Hohlfasern (3) nahe der Trennwände (41, 42) eine Packungsdichte $d_1$ von 20 bis 30% sowie nahe einem axialen Mittelpunkt eine Packungsdichte $d_2$ von 40 bis 50% aufweist, wobei das Verhältnis $d_1/d_2$ von 4/10 bis 6/10 beträgt, so daß der Druckverlust während des Blutdurchgangs aufgrund der Schwerkraft auf 60 mmHg begrenzt ist.

2. Oxygenator nach Anspruch 1, bei dem jede der Hohlfasern (3) eine aus einem Polyolefin bestehende poröse Hohlfaser ist, die einen Innendurchmesser von etwa 100 bis etwa 1000 µm, eine Wandstärke von etwa 10 bis etwa 200 µm, einen durchschnittlichen Porendurchmesser von etwa 200 bis etwa 2000 Å und eine Porosität von 20 bis 30% aufweist.

3. Oxygenator nach Anspruch 2, bei dem das Polyolefin Polypropylen ist.

4. Oxygenator nach Anspruch 1, bei dem die Wandung das Gehäuses (1) zu einem axialen Mittelpunkt hinderart verjüngt zuläuft, daß die Wandungsdicke einen minimalen Innendurchmesser ungefähr an dem Mittelpunkt aufweist und von dort aus auf die gegenüberligenden Endabschnitte konisch auseinanderläuft.

## Revendications

1. Oxygénateur (1) du type à fibres creuses, comprenant

une enveloppe (2), comportant des parties terminales opposées,

un faisceau (35), constitué d'une pluralité de fibres creuses (3), s'étendant axialement à travers l'envelopppe (2), chacune d'elles présentant une membrane à échange de gaz,

des cloisons (41, 42) fixées dans l'enveloppe (2) au niveau de ses parties terminales opposées, et retenant d'une manière étanche aux fluides les extrémités opposées des fibres creuses (3), sans colmater l'ouverture des fibres (3),

un orifice d'entrée de gaz (24) et un orifice de sortie de gaz (26), les deux étant en communication pour fluide avec l'espace intérieur desdites fibres creuses (3),

une chambre à sang (5) définie par les cloisons (41, 42), la surface extérieure desdites fibres creuses (3) et la surface intérieure de ladite enveloppe (2), et

un orifice d'entrée du sang (64) et un orifice de sortie du sang (62), disposés dans la paroi de l'enveloppe au niveau des parties terminales opposées (41, 42), en communication pour fluide avec ladite chambre à sang (5),

caractérisé par le fait que le faisceau (35) de fibres creuses (3) a une densité de remplissage d1 de 20 à 30% au voisinage des cloisons (41, 42) et une densité de remplissage d2 de 40 à 50% au voisinage d'un point central axial, le rapport d1/d2 étant compris entre 4/10 et 6/10, ce qui limite à 60 mmHg la chute de pression pendant le passage du sang par gravité.

2. Oxygénateur (1) selon la revendication 1, dans lequel chacune desdites fibres creuses (3) est une fibre creuse poreuse faite en une polyoléfine ayant un diamètre intérieur d'environ 100 à environ 1000 µm, une épaisseur de paroi d'environ 10 à environ 200 µm, un diamètre moyen de pores d'environ 200 à environ 2000 Å et une porosité de 20 à 80%.

3. Oxygénateur (1) selon la revendication 2, dans lequel la polyoléfine est le polypropylène.

4. Oxygénateur (1) selon la revendication 1, dans lequel la paroi de l'enveloppe (1) est effilée vers un point central axial de telle sorte que l'épaisseur de la paroi présente un diamètre intérieur minimal approximativement au point central, et, à partir de là, s'évase vers les parties terminales opposées.

# F I G. 1

Packing Density $d_1$

Gas In

21 24 23

62

Blood Out

41

3

5

1 2

35

61

Blood In

42

22 26 25

Gas Out

Packing Density $d_2$

$r_2$

1

# FIG. 2

# F I G. 3

# F I G. 4

Gas In

Gas Out

1

8

# F I G. 5

# F I G. 6

# F I G. 7

FIG. 8

FIG. 9